# EUROPEAN PATENT APPLICATION

(11) **EP 1 825 847 A2**
(43) Date of publication of application: **29.08.2007**
(21) Application number: 07250774.2
(22) Date of filing: 23.02.2007
(51) Int. Cl.: A61K 9/20, A61K 31/405, A61P 3/06

(54) **Fluvastatin sodium pharmaceutical compositions**

(30) Priority: 24.02.2006 US 776526 P
(71) Applicant: Teva Pharmaceutical Industries Ltd, 49131 Petah Tiqva (IL)
(72) Inventor: Di Capua, Simona, Kfar Saba (IL); Cohen, Yael Rotbart, Modiin 71700 (IL); Yaffeh, Ronit, Modiin (IL); Shterman, Nava, Petach Tikva 49214 (IL); Zilberman, Rina, Kfar Sava 44390 (IL)
(74) Representative: Russell, Tim

(57) **Abstract**

Various fluvastatin compositions and methods for preparing them are described. One example is a controlled release pharmaceutical composition comprising fluvastatin and at least one non-ionic hydrophilic polymer, wherein the composition is substantially free of hydroxypropyl methylcellulose. Another example is a stable pharmaceutical composition comprising fluvastatin, preferably, fluvastatin sodium wherein the composition is substantially free of an alkalizing stabilizing agent. Another example is a stable controlled release pharmaceutical formulation, comprising fluvastatin, preferably, fluvastatin sodium, that is stable with a water content greater than 3.5 percent by weight.

## Description

### Cross Reference to Related Applications

The present application claims benefit of U.S. Provisional Patent Application No. 60/776,526, filed February 24, 2006, the contents of which are incorporated herein by reference in their entirety.

### Field of Invention

The invention is directed to a controlled release pharmaceutical composition, comprising fluvastatin, preferably, fluvastatin sodium, and a hydrophilic polymer, where the controlled release pharmaceutical composition is substantially free of hydroxypropyl methylcellulose. The invention is further directed to a stable controlled release pharmaceutical formulation, comprising fluvastatin, preferably, fluvastatin sodium, where the stable formulation is substantially free of any alkaline stabilizing agent, such that the controlled release fluvastatin formulations of the invention are stable at a pH of less than 8. The invention is further directed to a stable controlled release pharmaceutical formulation, comprising fluvastatin, preferably, fluvastatin sodium, that is stable with a water content greater than 3.5 percent by weight.

### Background of the Invention

Fluvastatin has the chemical name [*R**,*S**-(*E*)]-(±)-7-[3-(4-fluorophenyl)-1-(1-methylethyl)-1*H*-indol-2-yl]-3,5-dihydroxy-6-heptenoic acid, and may be represented by chemical structure I:

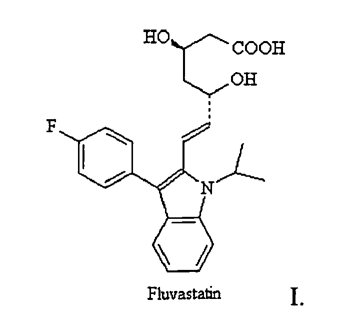

Fluvastatin sodium is a synthetic HMG-CoA reductase inhibitor, inhibiting 3-hydroxy-3-methylglutaryl-coenzyme A. Fluvastatin sodium is marketed by Novartis as LESCOL® and LESCOL XL®. The use of fluvastatin sodium therapeutically for lowering cholesterol has been reported, particularly in the treatment of hyperlipoproteinemia and atherosclerosis.

Controlled release tablet formulations of fluvastatin sodium are reported in U.S. Patent Application No. 10/100,656, published as U.S. Patent Application Publication No. 2002/0169145, and U.S. Patent No. 6,242,003. Reportedly, the disclosed tablets are administered once daily, yielding effective plasma levels over 12 hours.

U.S. Patent Application Publication No. 2002/0169145 A1 reports marketable dosage forms of formulations that comprise HMG-CoA reductase compounds containing hydroxypropyl methylcellulose together with non-ionic, hydrophilic polymers to prevent the premature release of significant amounts of the active agent. The non-ionic, hydrophilic polymers are reportedly selected from the group consisting hydroxyethylcellulose (MW about 90,000 to about 1,300,000), hydroxypropyl cellulose (MW about 370,000 to about 1,500,000) and poly(ethylene oxide) (MW about 100,000 to about 500,000).

U.S. Patent No. 6,242,003 reports the use of hydroxypropyl methylcellulose and fluvastatin, having a defined granule particle size, in order to achieve a color stable, sustained release formulation.

It is well known in the prior art, that many HMG-CoA reductase inhibitors, which are also known as statins, used in pharmaceutical compositions for treatment of hyperlipoproteinemia and atherosclerosis, are particularly susceptible to degradation at a pH of less than about 8. Therefore, such prior art pharmaceutical compositions further comprise an alkalizing stabilizing agent to maintain the pH, and avoid degradation of the HMG-CoA reductase inhibitor. Typically, HMG-CoA reductase inhibitors have also been found to be unstable in the presence of moisture and light, and, thus, such inhibitors were typically produced with a low water content.

U.S. Patents Nos. 5,356,896, 6,531,507, and 6,558,659 and U.S. Patent Application Publication No. 2003/0109584 A1 disclose various methods for stabilizing the acid labile statin compounds.

U.S. Patent No. 5,356,896, assigned to SANDOZ, discloses a reportedly stable pharmaceutical composition, comprising an alkaline stabilizing medium capable of imparting a pH of at least 8 to an aqueous solution or dispersion of the composition.

An alternative approach for stabilizing a HMG-CoA reductase inhibitor is reported in U.S. Patent No. 6,531,507, assigned to LEK Pharmaceuticals. In the reported method, the reductase inhibitor is stabilized by the co-crystallization or co-precipitation of the acid labile active ingredient with a buffering or basifying substance.

International Patent Application No. WO 2004/071402 discloses reportedly stable pharmaceutical dosage forms, comprising one or more active substances that are pH sensitive and one or more pharmaceutical excipients, where the water content is less than about 3.5 percent (w/w), and alkalizing or buffering substances or combinations thereof are not present.

### Summary of the Invention

The present invention provides pharmaceutical compositions, comprising an HMG-CoA reductase inhibitor, such as fluvastatin, and a hydrophilic polymer, substantially free of hydroxypropyl methylcellulose. With the compositions of the invention, the premature release of any significant amount of the active agent is substantially prevented. Preferred compositions of the invention comprising an HMG-CoA reductase inhibitor, such as fluvastatin, and a hydrophilic polymer, substantially free of hydroxypropyl methylcellulose, are stable in the absence of an alkalizing agent.

The present invention is directed to pharmaceutical compositions, preferably comprising a controlled release matrix system. The pharmaceutical compositions of the invention comprise fluvastatin, most preferably fluvastatin sodium, and a hydrophilic polymer, preferably a non-ionic hydrophilic polymer, where the hydrophilic polymer is not hydroxypropyl methylcellulose, and hydroxypropyl methylcellulose is not present in the compositions of the invention, except, possibly, in trace amounts, as an impurity that may possibly be present in a non-functional amount.

Preferably, the composition is a controlled release formulation of fluvastatin sodium, comprising about 10 to about 50 percent by weight of fluvastatin sodium, about 5 to about 40 percent by weight of a non-ionic hydrophilic polymer, about 20 to about 70 percent by weight of microcrystalline cellulose, about 0 to about 40 percent by weight of cross-linked polyvinyl pyrollidone, and about 0.5 to about 2 percent by weight of lubricant, where the composition is substantially free of hydroxypropyl methylcellulose.

In an alternative preferred embodiment, the composition is a controlled release formulation of fluvastatin or a salt thereof, consisting essentially of fluvastatin or a salt thereof, a hydrophilic polymer, preferably a non-ionic hydrophilic polymer, where the hydrophilic polymer is not hydroxypropyl methylcellulose, and optionally a filler, a lubricant, and/or a disintegrant.

In an alternative preferred embodiment, the composition is a controlled release formulation of fluvastatin or a salt thereof, consisting of fluvastatin or a salt thereof, a hydrophilic polymer, preferably a non-ionic hydrophilic polymer, where the hydrophilic polymer is not hydroxypropyl methylcellulose, and optionally a filler, a lubricant, and/or a disintegrant.

Preferably, the non-ionic hydrophilic polymer comprises at least one of poly(ethylene oxide), cellulose derivatives, such as carboxymethyl cellulose, methylcellulose, hydroxyethyl cellulose, and hydroxypropyl cellulose, polysaccharides, such as xanthan gum, inulin, guar gum, chitosan, certonia, carrageenan, starch, and starch derivatives, and combinations thereof.

More preferably, the non-ionic hydrophilic polymer is selected from the group of consisting of hydroxyethyl cellulose, having a molecular weight of from about 90,000 to about 1,300,000, hydroxypropyl cellulose, having a molecular weight of from about 80,000 to about 1,150,000, and poly(ethylene oxide), having a molecular weight of from about 100,000 to about 7,000,000. Most preferably, the non-ionic hydrophilic polymer is selected from the group of cellulose derivatives consisting of hydroxyethyl cellulose, having a molecular weight of from about 300,000 to about 1,000,000, hydroxypropyl cellulose, having a molecular weight of from about 370,000 to about 1,150,000, and poly(ethylene oxide), having a molecular weight of from about 1,000,000 to about 5,000,000.

A hydroxyethyl cellulose useful in the invention preferably has a viscosity in aqueous solution of from about 250 to about 6,500 mPas at a 2 percent concentration, from about 1,500 to about 5,500 mPas at a 1 percent concentration, and/or from about 75 to about 150 mPas at a 5 percent concentration. A hydroxypropyl cellulose useful in the invention preferably has a viscosity in aqueous solution of from about 150 to about 6,500 mPas at a 2 percent concentration, from about 1,500 to about 3,000 mPas at a 1 percent concentration, from about 75 to about 400 mPas at a 5 percent concentration, and/or from about 300 to about 600 mPas at a 10 percent concentration. A poly(ethylene oxide) useful in the invention preferably has a viscosity in aqueous solution of from about 400 to about 4,000 mPas at a 2 percent concentration, from about 1,650 to about 10,000 mPas at 1 percent concentration, and/or from about 30 to about 17,600 mPas at a 5 percent concentration.

The present invention provides a stable pharmaceutical controlled release formulation, comprising an HMG-CoA reductase inhibitor, preferably fluvastatin, and, more preferably, fluvastatin sodium, where the formulation is stable with a water content of greater than 3.5 percent by weight.

The present invention provides a stable pharmaceutical controlled release formulation, comprising an HMG-CoA reductase inhibitor, preferably fluvastatin, and, more preferably, fluvastatin sodium, without requiring stabilization with a basic environment. That is, the HMG-CoA reductase inhibitor formulations of the invention, which preferably comprise fluvastatin sodium, are stable without the addition of any alkaline stabilizing medium or alkalizing agent. Preferably, such HMG-CoA reductase inhibitor formulations of the invention are stable with a relatively high water content. In particular, HMG-CoA reductase inhibitor formulations of the invention, having a water content greater than about 3.5 percent, are stable.

### Brief Description of the Drawing

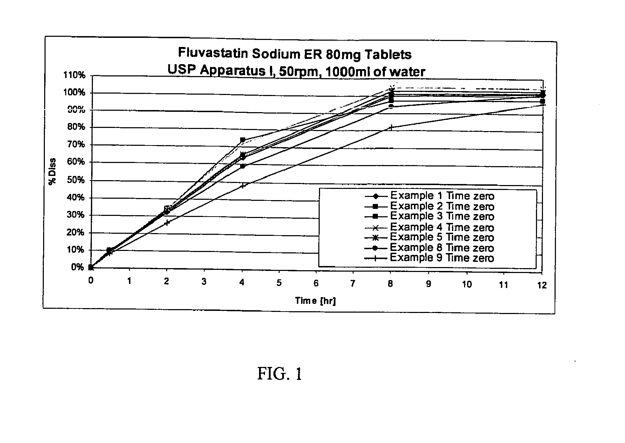

Figure 1 illustrates the dissolution profiles of controlled release compositions of the invention comprising fluvastatin sodium.

### Detailed Description of the invention:

Although the invention is described primarily herein in terms of fluvastatin, preferably, fluvastatin sodium, one of ordinary skill in the art will recognize that the present invention is also useful with compositions and formulations of other HMG-CoA reductase inhibitors.

As used herein, the term "fluvastatin" refers to the compound fluvastatin and pharmaceutically acceptable salts thereof. It also encompasses all solid forms of fluvastatin and its salts, including amorphous forms, polymorphs, hydrates, and solvates. A preferred salt of fluvastatin is fluvastatin sodium.

As used herein, the terms "stable fluvastatin" and "stable HMG-CoA reductase inhibitor" refer to fluvastatin or HMG-CoA reductase inhibitor, having:
a) an assay of more than about 95 percent of the stable fluvastatin or HMG-CoA reductase inhibitor, following storage for three months at a temperature of 40°C and a relative humidity of 75 percent;
b) an impurities degradation profile of less than about 1 percent of the total weight of the composition of fluvastatin sodium anti-isomer, following storage for three months at a temperature of 40°C and a relative humidity of 75 percent;
c) an impurities degradation profile of less than about 1 percent of the total weight of the composition of fluvastatin hydroxyl diene, following storage for three months at a temperature of 40°C and a relative humidity of 75 percent; and/or
d) an impurities degradation profile of less than about 0.2 percent of the total weight of the composition of total degradation impurities (other than fluvastatin sodium anti-isomer and fluvastatin hydroxyl diene), following storage for three months at a temperature of 40°C and a relative humidity of 75 percent.

As used herein, the terms "alkaline stabilizing agent" and "alkalizing agent" refer to a pharmaceutical excipient that, when combined with all other excipients, if any, in a given composition imparts a pH of 8, or greater, to an aqueous solution or dispersion of the composition. A composition of the invention that is "substantially free of" alkaline stabilizing agents and alkalizing agents contains such agents only in trace amounts as an impurity. That is, in compositions of the invention that are "substantially free of" alkaline stabilizing agents and alkalizing agents, such agents are not present in the compositions of the invention, except, possibly, as a trace impurity that may possibly be present in a non-functional amount. Compositions and formulations of the invention that are substantially free of any alkaline stabilizing agent or alkalizing agent preferably comprise a mixture of excipients that provide a pH of less than 8.

As used herein, the term "water content" refers to the content of water based upon the Loss on Drying method (the "LOD" method).

The present invention provides controlled-release pharmaceutical formulations, comprising fluvastatin and at least one hydrophilic polymer, preferably a non-ionic hydrophilic polymer, where the formulation is substantially free of hydroxypropyl methylcellulose (HPMC). That is, the non-ionic hydrophilic polymer is not hydroxypropyl methylcellulose, and hydroxypropyl methylcellulose is not present in the compositions of the invention, except, possibly, as a trace impurity that may possibly be present in a non-functional amount.

The present invention also provides stable, controlled-release pharmaceutical formulations, comprising fluvastatin, in the absence of an alkaline stabilizing agent.

The present invention also provides stable, controlled-release pharmaceutical formulations, comprising fluvastatin, having a relatively high water content; preferably the water content is greater than about 3.5 percent.

Preferably, formulations of the invention that comprise a hydrophilic polymer and are substantially free of hydroxypropyl methylcellulose are stable in the absence of an alkaline stabilizing agent.

Preferably, formulations of the invention that comprise a hydrophilic polymer and are substantially free of hydroxypropyl methylcellulose are stable with a water content of greater than about 3.5 percent by weight.

The preferred form of fluvastatin is fluvastatin sodium. The fluvastatin may be preferably amorphous, crystalline, or a combination thereof. Preferably, the controlled-release pharmaceutical formulations of the invention comprise from about 10 to about 50 percent by weight of fluvastatin, such as fluvastatin sodium, more preferably, from about 10 to about 40 percent by weight, and, most preferably, from about 15 to about 35 percent by weight.

Preferably, formulations of the invention that comprise a hydrophilic polymer and are substantially free of hydroxypropyl methylcellulose comprise from about 5 to about 40 percent by weight of a hydrophilic polymer, preferably, a non-ionic hydrophilic polymer, more preferably, from about 10 to about 35 percent by weight, and, most preferably, from about 10 to about 30 percent by weight.

Hydrophilic polymers useful in the invention include, but are not limited to, non-ionic hydrophilic polymers, except the non-ionic hydrophilic polymer is not hydroxypropyl methylcellulose. Preferred non-hydroxypropyl methylcellulose hydrophilic polymers include cellulose derivatives. Preferably, a cellulose derivative useful in the invention has a molecular weight in the range of from about 80,000 to about 1,300,000 Daltons, and, most preferably, from about 300,000 to about 1,150,000 Daltons. Preferred non-ionic hydrophilic polymers also include poly(ethylene oxide) polymers. Poly(ethylene oxide) polymers useful in the invention preferably have a molecular weight in the range of from about 100,000 to about 7,000,000, and, more preferably, from about 1,000,000 to about 5,000,000.

Preferably, the non-ionic hydrophilic polymer useful in the invention has a viscosity in a 2 percent by weight aqueous solution of from about 150 to about 6500 mPas, and, more preferably, from about 2000 to about 6500 mPas.

Preferably, the non-ionic hydrophilic polymer useful in the invention has a viscosity in a 1 percent by weight aqueous solution of from about 1500 to about 10,000 mPas, and, more preferably, from about 1500 to about 7500 mPas.

Preferably, the non-ionic hydrophilic polymer is hydroxyethyl cellulose, hydroxypropyl cellulose, poly(ethylene oxide), or a mixture thereof.

Preferably, hydroxyethyl cellulose polymers useful in the invention preferably have a molecular weight in the range of from about 90,000 Daltons to about 1,300,000 Daltons, and, more preferably, from about 300,000 Daltons to about 1,000,000 Daltons. Preferably, hydroxyethyl cellulose polymers useful in the invention have a viscosity of from about 250 to about 6,500 mPas, and, more preferably, from about 4,500 to about 6,500 mPas in a 2 percent aqueous solution. Preferably, a hydroxyethyl cellulose polymer useful in the invention has a viscosity from about 1,500 to about 5,500 mPas, and, more preferably, from about 1,500 to about 2,500 mPas in a 1 percent solution.

Preferably, hydroxypropyl cellulose polymers useful in the invention have a molecular weight in the range of from about 80,000 Daltons to about 1,150,000 Daltons, and, more preferably, from about 370,000 Daltons to about 1,150,000 Daltons. Preferably, hydroxypropyl cellulose polymers useful in the invention have a viscosity of from about 150 to about 6,500 mPas, and, more preferably, from about 4,000 to about 6,500 mPas in a 2 percent solution. Preferably, hydroxypropyl cellulose polymers useful in the invention have a viscosity of from about 1,500 to about 3,000 mPas in a 1 percent solution.

Preferably, poly(ethylene oxide) polymers useful in the invention have a molecular weight in the range of from about 100,000 Daltons to about 7,000,000 Daltons, and, more preferably, from about 1,000,000 Daltons to about 5,000,000 Daltons.

Preferably, a 2 percent solution of poly(ethylene oxide) has viscosity from about 400 to about 4,000 mPas, and, more preferably, from about 2,000 to about 4,000 mPa. Preferably, a 1 percent solution of poly(ethylene oxide) has a viscosity of from about 1,650 to about 10,000 mPas, and, more preferably, from about 1,650 to about 7,500 mPas.

The formulations of the present invention may comprise one or more additional excipients, such as disintegrants, binders, fillers, lubricants, and surfactants, where the additional excipient is not hydroxypropyl methylcellulose. Preferred disintegrants include, but are not limited to, carboxymethylcellulose sodium, carboxymethylcellulose calcium, croscarmellose sodium, cross-linked polyvinyl pyrollidone, starch, polacrilin potassium, hydroxypropyl cellulose low substituted, powdered cellulose, and povidone. Cross-linked polyvinyl pyrollidone is particularly preferred.

Preferred fillers include, but are not limited to, microcrystalline cellulose, lactose, starch, manitol, cellulose, sorbitol, and dibasic calcium phosphate. Microcrystalline cellulose is particularly preferred.

Preferred surfactants include, but are not limited to, sodium lauryl sulfate, docusate sodium, glyceryl monooleate, and cetrimide. Sodium lauryl sulfate is particularly preferred.

Preferably, a formulation of the invention comprises at least one hydrophilic excipient in addition to the hydrophilic polymer. Useful hydrophilic excipients include, but are not limited to microcrystalline cellulose, lactose, manitol, reducing sugars, non-reducing sugars, and cross-linked polyvinyl pyrollidone. Microcrystalline cellulose and cross-linked polyvinyl pyrollidone are particularly preferred.

Preferably, a formulation of the invention comprises from about 20 to about 70 percent by weight of a hydrophilic excipient, more preferably, from about 25 to about 65 percent by weight, and, most preferably, from about 30 to about 60 percent by weight.

Preferably, a formulation of the invention comprises from about 10 to about 50 percent by weight fluvastatin sodium, from about 5 to about 40 percent by weight of one or more non-ionic hydrophilic polymers, excluding hydroxypropyl methylcellulose, from about 20 to about 70 percent by weight microcrystalline cellulose, from about 0 to about 40 percent cross-linked polyvinyl pyrollidone, and from about 0.5 to about 2 percent of a lubricant.

Preferred controlled-release formulations of the present invention are stable in the absence of an alkaline stabilizing agent. Such formulations preferably comprise a mixture of excipients that impart a pH of less than 8 to an aqueous suspension or solution of the composition.

Preferred controlled-release formulations of the invention have been found to be stable even with a water content of greater than about 3.5 percent by weight of the formulation.

The stability of fluvastatin sodium formulations in accordance with the present invention were monitored, according to the pharmaceutical industry standard, under accelerated storage conditions of about 40°C and about 75 percent relative humidity for three months. The final preparations demonstrated satisfactory stability for the formulations under those conditions. Preferably, after three months of storage under such conditions, an oral dosage form in accordance with the invention has an assay purity of more than about 95 percent, more preferably about 97 percent, and most preferably about 100 percent, as compared to the label claimed dosage of pure fluvastatin sodium. The results of these stability measurements are set forth below in Table 12.

Moreover, after three months of storage under such conditions, an oral dosage form in accordance with the invention preferably has an impurities degradation profile of less than about 1 percent of fluvastatin sodium anti-isomer, and, more preferably, less than about 0.5 percent by weight. The structural formula of the fluvastatin sodium anti-isomer is

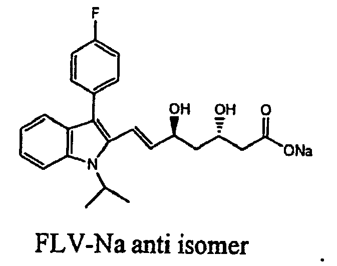

Furthermore, after three months of storage under such conditions, an oral dosage form in accordance with the invention preferably has an impurities degradation profile of less than about 1 percent of fluvastatin hydroxyl diene, and, more preferably, less than about 0.5 percent by weight. The structural formula of fluvastatin hydroxyl diene is

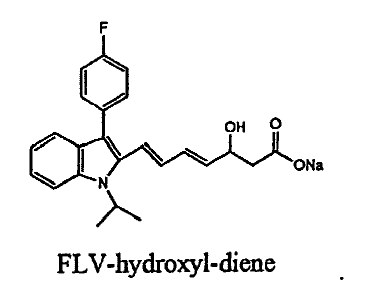

Preferably, a controlled-release formulation in accordance with the present invention will gradually release greater than about 80 percent of the drug within a time period of about 12 hours, under dissolution conditions of USP Apparatus I (basket), rotational speed: 50 rpm, medium: water at 37°C, and Volume: 1000 ml. Alternatively, a formulation in accordance with the present invention will release greater than about 90 percent, preferably greater than about 95 percent, of the drug within a time period of about 12 hours under the same conditions.

The present invention also provide a process for preparing the above controlled release pharmaceutical composition, comprising combining fluvastatin or a salt thereof with at least one hydrophilic polymer, wherein the hydrophilic polymer is not hydroxypropyl methylcellulose.

The present invention also provides a method for manufacturing the above controlled-release formulation. The method preferably comprises the steps of granulation in a high shear mixer followed by drying in a fluidized bed. The formed granules, after drying, are preferably combined with extra-granular excipients, and then compressed into tablets. Preferably, the last step of the manufacturing procedure is the tablet coating process for cosmetic purposes.

The following non-limiting examples are merely illustrative of the preferred embodiments of the present invention, and are not to be construed as limiting the invention, the scope of which is defined by the appended claims.

### Example 1

**Table 1**

| **Ingredient** | **Amount (mg/tablet)** |
|---|---|
| Microcrystalline Cellulose | 146.44 |
| Fluvastatin Sodium | 84.24 |
| Hydroxyethyl cellulose | 50.00 |
| Magnesium Stearate | 3.00 |
| Total | 283.68 |

Method of manufacturing: microcrystalline cellulose, fluvastatin sodium, and hydroxyethyl cellulose were transferred into a high shear mixer, and granulated using alcohol. The granulated mixture was then dried in a fluid bed dryer using a target inlet temperature of 50°C until the outlet temperature reached 35°C. Then, the dried granules were passed through a 0.8 mm screen using an oscillating mill. The milled granules and microcrystalline cellulose were dry blended in a mixer. Magnesium stearate was prescreened through a 50 mesh screen, and then blended in a mixer. The final granulation blend was then compressed into tablets.

### Example 2

**Table 2**

| **Ingredient** | **Amount (mg/tablet)** |
|---|---|
| Microcrystalline Cellulose | 146.44 |
| Fluvastatin Sodium | 84.24 |
| Cross-linked polyvinyl pyrollidone | 60.00 |
| Hydroxyethyl cellulose | 50.00 |
| Magnesium Stearate | 3.00 |
| Total | 343.68 |

Method of manufacturing: microcrystalline cellulose, fluvastatin sodium, cross-linked polyvinyl pyrollidone, and hydroxyethyl cellulose were transferred into a high shear mixer, and granulated using alcohol. The granulated mixture was dried in a fluid bed dryer at a target inlet temperature of 50°C until the outlet temperature reached 35°C. The dried granules were passed through a 0.8 mm screen using an oscillating mill. The milled granules and microcrystalline cellulose were dry blended in a mixer. Magnesium stearate was prescreened through a 50 mesh screen, and then blended in a mixer. The final granulation blend was then compressed into tablets.

### Example 3

**Table 3**

| **Ingredient** | **Amount (mg/tablet)** |
|---|---|
| Microcrystalline Cellulose | 146.44 |
| Fluvastatin Sodium | 84.24 |
| Hydroxyethyl cellulose NF (Natrosol 250M Pharm) | 50.00 |
| Sodium Lauryl Sulfate | 7.00 |
| Magnesium Stearate | 3.00 |
| Total | 290.68 |

Method of manufacturing: microcrystalline cellulose fluvastatin sodium, hydroxyethyl cellulose, and sodium lauryl sulfate were transferred into a high shear mixer, and granulated using alcohol. The granulated mixture was then dried in a fluid bed dryer at a target inlet temperature of 50°C until the outlet temperature reached 35°C. Then, the dried granules were passed through a 0.8 mm screen using an oscillating mill. The milled granules and microcrystalline cellulose were dry blended in a mixer. Magnesium stearate was prescreened through a 50 mesh screen, and then blended in a mixer. The granulation final blend was then compressed into tablets.

### Example 4

**Table 4**

| **Ingredient** | **Amount (mg/tablet)** |
|---|---|
| Microcrystalline Cellulose | 146.44 |
| Fluvastatin Sodium | 84.24 |
| Cross-linked polyvinyl pyrollidone | 90.00 |
| Hydroxyethyl cellulose | 50.00 |
| Magnesium Stearate | 3.00 |
| Total | 373.68 |

Method of Manufacturing: Same as for Example 2.

### Example 5

**Table 5**

| **Ingredient** | **Amount (mg/tablet)** |
|---|---|
| Microcrystalline Cellulose | 145.43 |
| Cross-linked polyvinyl pyrollidone | 30.00 |
| Fluvastatin Sodium | 84.24 |
| Hydroxyethyl cellulose | 113.00 |
| Magnesium Stearate | 3.00 |
| Total | 375.67 |

Method of Manufacturing: Same as for Example 2.

### Example 6

**Table 6**

| **Ingredient** | **Amount (mg/tablet)** |
|---|---|
| Microcrystalline Cellulose | 146.00 |
| Fluvastatin Sodium | 84.24 |
| Hydroxyethyl cellulose | 50.00 |
| Magnesium Stearate | 3.00 |
| *OPADRY II (high performance) white | 9.00 |
| Total | 292.24 |

| | |
|---|---|
| * A commercially available powder mix for dispersion composed of polyvinyl alcohol - part hydrolyzed, Titanium dioxide, Macrogol / PEG 3350 and Talc. | |

Method of manufacturing: Same as for example 1, except for film coating of compressed tablets.

Coating method: OPADRY II (high performance) White was mixed with the required quantity of purified water to obtain a 20 percent w/w suspension. The tablets were transferred to a coating pan, and pre-warmed to about 35° to about 65°C. The OPADRY II (high performance) suspension was sprayed until a 3 to 5 percent w/w solid weight gain per tablet was achieved.

### Example 7

**Table 7**

| **Ingredient** | **Amount (mg/tablet)** |
|---|---|
| Microcrystalline Cellulose | 146.00 |
| Cross-linked polyvinyl pyrollidone | 30.00 |
| Fluvastatin Sodium | 84.24 |
| Hydroxyethyl cellulose | 50.00 |
| Magnesium Stearate | 3.00 |
| OPADRY II (high performance) white | 9.70 |
| Total | 322.94 |

Method of manufacturing: Same as example 2. Method of manufacturing film coated compressed tablets: Same as example 6.

### Example 8

**Table 8**

| **Ingredient** | **Amount (mg/tablet)** |
|---|---|
| Microcrystalline Cellulose | 146.00 |
| Cross-linked polyvinyl pyrollidone | 70.00 |
| Fluvastatin Sodium | 84.24 |
| Hydroxyethyl cellulose | 50.00 |
| Magnesium Stearate | 3.00 |
| OPADRY II (high performance) white | 17.76 |
| Total | 371.00 |

Method of Manufacturing: Same as for example 7.

### Example 9

**Table 9**

| **Ingredient** | **Amount (mg/tablet)** |
|---|---|
| Microcrystalline Cellulose | 146.00 |
| Cross-linked polyvinyl pyrollidone | 30.0 |
| Fluvastatin Sodium | 84.24 |
| Hydroxyethyl cellulose | 70.00 |
| Magnesium Stearate | 3.00 |
| OPADRY II (high performance) white | 16.76 |
| Total | 350.00 |

Method of Manufacturing: Same as for example 7.

### Example 10

**Table 10**

| **Ingredient** | **Amount (mg/tablet)** |
|---|---|
| Microcrystalline Cellulose | 146.00 |
| Cross-linked polyvinyl pyrollidone | 30.00 |
| Fluvastatin Sodium | 84.24 |
| Hydroxyethyl cellulose | 65.00 |
| Magnesium Stearate | 3.00 |
| OPADRY II (high performance) white | 16.76 |
| Total | 345.00 |

Method of Manufacturing: Same as for example 7.

### Example 11

**Table 11**

| **Ingredient** | **Amount (mg/tablet)** |
|---|---|
| Microcrystalline Cellulose | 146.00 |
| Cross-linked polyvinyl pyrollidone | 30.00 |
| Fluvastatin Sodium | 84.24 |
| Hydroxyethyl cellulose | 68.00 |
| Magnesium Stearate | 3.00 |
| OPADRY II (high performance) white | 16.76 |
| Total | 348.00 |

Method of Manufacturing: Same as for example 7.

Stability data for the products of Examples 1, 7, 10, and 11 are set forth in Table 12. (The stability of products of examples 2 to 6, 8, and 9 was not tested).

### Stability Data

**Table 12**

| | Time Zero | | | 3 month stability, [40°C, 75%RH] | | |
|---|---|---|---|---|---|---|
| | Assay [%] | IDD* [%] | | Assay [%] | IDD* [%] | |
| | | Anti isomer | Fluvastatin hydroxyl diene | | Anti isomer | Fluvastatin hydroxyl diene |
| Example 1 | 98.7 | 0.1 | - | 102.4 | 0.2 | - |
| Example 7 | 101.6 | 0.1 | <0.1 | 100.9 | 0.3 | <0.1 |
| Example 10 | 98.6 | 0.1 | <0.1 | 97.9 | 0.3 | 0.2 |
| Example | 96.0 | <0.1 | <0.1 | 95.2 | 0.3 | 0.1 |
| LESCOL XL^{®} | 97.7 | 0.2 | <0.1 | 97.3 | 0.3 | 0.4 |

| | | | | | | |
|---|---|---|---|---|---|---|
| *Impurity and Degradation Determination. | | | | | | |

Preferably, a composition of the invention contains a level of impurities and degradation products other than fluvastatin sodium anti-isomer and fluvastatin hydroxyl diene of no more than about 0.2 percent. All analyzed samples of formulations of the invention were found to have a level of impurities and degradation products other than fluvastatin sodium anti-isomer and fluvastatin hydroxyl diene of less than about 0.1 percent, at Time Zero and after 3 months of storage under accelerated conditions of a temperature of about 40°C and a relative humidity of about 75 percent. No fluvastatin lactone impurity was detected in any of the samples of compositions of the invention that were analyzed.

The total water content, as measured by Loss on Dry (LOD), of the analyzed compositions is provided in Table 13. The total water content, as measured by Loss on Dry (LOD) was measured for the compositions of examples 6 to 11. The LOD was measured with a Mettler Toledo HR73 Moisture Content Analyzer at a temperature of 105°C, using Mode 3.

**Table 13**

| | |
|---|---|
| Example 6 | 4.0% |
| Example 7 | 4.1 % |
| Example 8 | 4.8% |
| Example 9 | 4.9% |
| Example 10 | 4.3% |
| Example 11 | 5.0% |

The data for the dissolution profiles of Examples 1 to 3 and 6 to 11 are provided in Table 14, and are illustrated in Figure 1. The dissolution method conforms to USP apparatus I: 50 rpm and 1000 ml of water. As set forth in Table 14, "Average [%]" refers to the mean result of the percentage of the active pharmaceutical ingredient released from the composition after a given amount of time has elapsed.

**Table 14**

| | Example 1 | | Example 2 | | Example 3 | | Example 6 | | Example 7 | |
|---|---|---|---|---|---|---|---|---|---|---|
| | 6 tablets | | 6 tablets | | 6 tablets | | 12 tablets | | 12 tablets | |
| Time [hr] | Average [%] | -/+ [%] | Average [%] | -/+ [%] | Average [%] | -/+ [%] | Average [%] | -/+ [%] | Average [%] | -/+ [%] |
| 0 | 0 | 0 | 0 | 0 | 0 | 0 | 0 | 0 | 0 | 0 |
| 0.5 | 9 | 8-9 | 11 | 9-12 | 9 | 8-9 | 10 | 9-44 | 9 | 6-12 |
| 2 | 33 | 28-35 | 34 | 29-40 | 33 | 30-35 | 34 | 30-38 | 33 | 25-46 |
| 4 | 66 | 57-67 | 74 | 70-80 | 65 | 63-68 | 72 | 61-78 | 66 | 56-81 |
| 8 | 100 | 97-101 | 97 | 96-98 | 101 | 100-101 | 105 | 101-108 | 103 | 98-106 |
| 10 | 101 | 101-102 | 97 | 97-98 | 101 | 100-102 | - | - | - | - |
| 12 | 101 | 101-102 | 98 | 97-98 | 102 | 101-104 | 105 | 101-108 | 103 | 98-106 |

| | Example 8 | | Example 9 | | Example 10 | | Example 11 | | LESCOL XL® | |
|---|---|---|---|---|---|---|---|---|---|---|
| | 6 tablets | | 6 tablets | | 12 tablets | | 12 tablets | | 12 tablets | |
| Time [hr] | Average [%] | -/+ [%] | Average [%] | -/+ [%] | Average [%] | -/+ [%] | Average [%] | -/+ [%] | Average [%] | -/+ [%] |
| 0 | 0 | 0 | 0 | 0 | 0 | 0 | 0 | 0 | 0 | 0 |
| 0.5 | 10 | 2-12 | 8 | 7-9 | 7 | 6-7 | 6 | 6-7 | 6 | 6-7 |
| 2 | 32 | 26-35 | 26 | 23-29 | 22 | 21-24 | 20 | 19-22 | 20 | 18-23 |
| 4 | 59 | 44-67 | 48 | 38-55 | 41 | 38-44 | 36 | 34-40 | 46 | 42-55 |
| 8 | 94 | 89-101 | 82 | 67-91 | 80 | 78-84 | 72 | 68-73 | 94 | 92-98 |
| 10 | - | - | - | - | 91 | 88-95 | - | - | - | - |
| 12 | 101 | 99-103 | 96 | 85-99 | - | - | 97 | 93-99 | 97 | 94-99 |

While it is apparent that the invention disclosed herein is well calculated to fulfill the objects stated above, it will be appreciated that numerous modifications and embodiments may be devised by those skilled in the art. Therefore, it is intended that the appended claims cover all such modifications and embodiments as falling within the true spirit and scope of the present invention.

## Claims

1. A controlled release pharmaceutical composition, comprising fluvastatin or a salt thereof and at least one hydrophilic polymer, wherein the hydrophilic polymer is not hydroxypropyl methylcellulose, and the composition is substantially free of hydroxypropyl methylcellulose.

2. The composition according to claim 1, wherein the hydrophilic polymer comprises a non-ionic hydrophilic polymer.

3. The composition according to either of claims 1 and 2, wherein the composition is substantially free of an alkalizing stabilizing agent.

4. The composition according to any of claims 1 to 3, wherein the salt of fluvastatin is fluvastatin sodium.

5. The composition according to any of claims 1 to 4, wherein the fluvastatin or a salt thereof comprises a hydrate, solvate, or amorphous form of fluvastatin.

6. The composition according to any of claims 1 to 5, wherein the fluvastatin or a salt thereof is present in an amount of from about 10 to about 50 percent by weight of the composition.

7. The composition according to any of claims 1 to 6, wherein the at least one hydrophilic polymer is present in an amount of from about 5 to about 40 percent by weight of the composition.

8. The composition according to any of claims 1 to 7, wherein the at least one hydrophilic polymer is selected from the group consisting of cellulose derivatives, poly(ethylene oxide), polysaccharides, and combinations thereof.

9. The composition according to claim 8, wherein the cellulose derivative is selected from the group consisting of carboxymethyl cellulose, methyl cellulose, hydroxyethyl cellulose, hydroxypropyl cellulose, and combinations thereof.

10. The composition according to claim 8, wherein the polysaccharide is selected from the group consisting of xanthan gum, inulin, guar gum, chitosan, certonia, carrageenan, starch, starch derivatives, and combinations thereof.

11. The composition according to claim 8, wherein the at least one hydrophilic polymer comprises a cellulose derivative having a molecular weight of from about 80,000 Daltons to about 1,300,000 Daltons, a poly(ethylene oxide) polymer having a molecular weight of from about 100,000 Daltons to about 7,000,000 Daltons, or a mixture thereof.

12. The composition according to claim 8, wherein the at least one hydrophilic polymer comprises hydroxyethyl cellulose having a molecular weight of from about 90,000 Daltons to about 1,300,000 Daltons.

13. The composition according to claim 8, wherein the at least one hydrophilic polymer comprises hydroxyethyl cellulose having a molecular weight of from about 300,000 Daltons to about 1,000,000 Daltons.

14. The composition according to any of claims 8, 12, and 13, wherein the at least one hydrophilic polymer comprises hydroxyethyl cellulose having a viscosity in a 2 percent by weight aqueous solution of from about 250 to about 6,500 mPas.

15. The composition according to any of claims 8, 12, and 13, wherein the at least one hydrophilic polymer comprises hydroxyethyl cellulose having a viscosity in a 1 percent by weight aqueous solution of from about 1,500 to about 5,500 mPas.

16. The composition according to claim 8, wherein the at least one hydrophilic polymer comprises hydroxypropyl cellulose having a molecular weight of from about 80,000 Daltons to about 1,150,000 Daltons.

17. The composition according to claim 8, wherein the at least one hydrophilic polymer comprises hydroxypropyl cellulose having a molecular weight of from about 370,000 Daltons to about 1,150,000 Daltons.

18. The composition according to any of claims 8, 16, and 17, wherein the at least one hydrophilic polymer comprises hydroxypropyl cellulose having a viscosity in a 2 percent by weight aqueous solution of from about 150 to about 6,500 mPas.

19. The composition according to any of claims 8, 16, and 17, wherein the at least one hydrophilic polymer comprises hydroxypropyl cellulose having a viscosity in a 1 percent by weight aqueous solution of from about 1,500 to about 3,000 mPas.

20. The composition according to claim 8, wherein the at least one hydrophilic polymer comprises poly(ethylene oxide) having a molecular weight of from about 1,000,000 Daltons to about 5,000,000 Daltons.

21. The composition according to claim 8, wherein the at least one hydrophilic polymer comprises poly(ethylene oxide) having a viscosity in a 2 percent by weight aqueous solution of from about 400 to about 4,000 mPas.

22. The composition according to any of claims 8, 20, and 21, wherein the at least one hydrophilic polymer comprises poly(ethylene oxide) having a viscosity in a 1 percent by weight aqueous solution of from about 1,650 to about 7,500 mPas.

23. The composition according to any of claims 1 to 22, further comprising at least one excipient selected from a group comprising of disintegrants, binders, fillers, lubricants, and surfactants.

24. The composition according to claim 23, wherein the excipient comprises at least one of:
a disintegrant selected from the group consisting of carboxymethylcellulose sodium, carboxymethylcellulose calcium, croscarmellose sodium, cross-linked polyvinyl pyrollidone, starch, polacrilin potassium, hydroxypropyl cellulose low substituted, powdered cellulose, and povidone;
a filler selected from the group consisting of microcrystalline cellulose, lactose, starch, manitol, cellulose, sorbitol, and dibasic calcium phosphate; and
a surfactant selected from the group consisting of sodium lauryl sulfate, docusate sodium, glyceryl monooleate, and cetrimide.

25. The composition according to any of claims 1 to 22, further comprising at least one hydrophilic excipient.

26. The composition according to claim 25, wherein the hydrophilic excipient is selected from the group consisting of starch, microcrystalline cellulose, cross-linked polyvinyl pyrollidone, lactose, manitol, reducing sugars and non-reducing sugars.

27. The composition according to any of claims 1 to 22, wherein the fluvastatin is present in an amount of from about 10 to about 50 percent by weight, the at least one hydrophilic polymer is present in an amount of from about 5 to about 40 percent, and the controlled-release composition further comprises from about 20 to about 70 percent microcrystalline cellulose, from 0 to about 40 percent cross-linked polyvinyl pyrollidone, and from about 0.5 to about 2 percent of a lubricant.

28. The composition according to claim 27, wherein the composition releases up to 80 percent of the fluvastatin within a time period of about 12 hours, when placed in a basket, having a rotational speed of about 50 rpm, in 1000 ml of water, having a temperature of 37°C.

29. The composition according to any of claims 1 to 7 and 23 to 28, wherein the hydrophilic polymer is selected from the group consisting of polymers having a viscosity in a 2 percent by weight aqueous solution of from about 150 to about 6,500 mPas, polymers having a viscosity in a 1 percent by weight aqueous solution of from about 1,650 to about 10,000 mPas, and mixtures thereof, wherein the composition is substantially free of hydroxypropyl methylcellulose.

30. A process for preparing a controlled release pharmaceutical composition according to any of claims 1 to 29, comprising combining fluvastatin or a salt thereof with at least one hydrophilic polymer, wherein the hydrophilic polymer is not hydroxypropyl methylcellulose.

31. A method of manufacturing the composition according to any of claims 1 to 7 and 23 to 28, comprising combining fluvastatin and the hydrophilic polymer, wherein the hydrophilic polymer is selected from the group consisting of polymers having a viscosity in a 2 percent by weight aqueous solution of from about 150 to about 6,500 mPas, polymers having a viscosity in a 1 percent by weight aqueous solution of from about 1,650 to about 10,000 mPas, and mixtures thereof.

32. A stable controlled-release pharmaceutical composition, comprising fluvastatin or a salt thereof, wherein the composition is substantially free of an alkalizing stabilizing agent.

33. The composition according to claim 32, further comprising at least one hydrophilic polymer, wherein the hydrophilic polymer is not hydroxypropyl methylcellulose, and the composition is substantially free of hydroxypropyl methyl cellulose, preferably the hydrophilic polymer is a non-ionic hydrophilic polymer.

34. The composition according to claim 33, wherein the fluvastatin or a salt thereof is present in an amount of from about 10 to about 50 percent by weight, the at least one hydrophilic polymer is present in an amount of from about 5 to about 40 percent, and the controlled-release composition further comprises from about 20 to about 70 percent microcrystalline cellulose, from 0 to about 40 percent cross-linked polyvinyl pyrollidone, and from about 0.5 to about 2 percent of a lubricant.

35. The composition according to any of claims 32 to 34, having a water content of greater than 3.5 percent.

36. The composition according to either of claims 33 and 35, wherein the fluvastatin or a salt thereof is present in an amount of from about 10 to about 50 percent by weight, the at least one hydrophilic polymer is present in an amount of from about 5 to about 40 percent, and the controlled-release composition further comprises from about 20 to about 70 percent microcrystalline cellulose, from 0 to about 40 percent cross-linked polyvinyl pyrollidone, and from about 0.5 to about 2 percent of a lubricant.

37. The composition according to 32, further comprising at least one hydrophilic polymer, wherein the hydrophilic polymer is not hydroxypropyl methylcellulose, and the composition is substantially free of hydroxypropyl methyl cellulose, wherein the composition is stable at a water content of greater than 3.5 percent, preferably the hydrophilic polymer is a non-ionic hydrophilic polymer.

38. The composition according to claim 37, wherein the fluvastatin or a salt thereof is present in an amount of from about 10 to about 50 percent by weight, the at least one hydrophilic polymer is present in an amount of from about 5 to about 40 percent, and the controlled-release composition further comprises from about 20 to about 70 percent microcrystalline cellulose, from 0 to about 40 percent cross-linked polyvinyl pyrollidone, and from about 0.5 to about 2 percent of a lubricant.

39. The composition according to any of claims 32, 33, 35, and 37, comprising from about 10 to about 50 percent (w/w %) of fluvastatin or a salt therof.

40. The composition according to any of claims 32 to 39, wherein the salt of fluvastatin is fluvastatin sodium.

41. The composition according to any of claims 32 to 40, wherein the fluvastatin or salt thereof in a solid form.

42. The stable controlled release pharmaceutical composition according to any of claims 32 to 41, wherein the fluvastatin or a salt thereof has an assay purity of more than about 95 percent by weight.

43. The stable controlled release pharmaceutical composition according to any of claims 32 to 41, wherein the fluvastatin or a salt thereof has an assay purity of more than about 95 percent, following storage for three months at a temperature of 40°C and a relative humidity of 75 percent.

44. The stable controlled release pharmaceutical composition according to any of claims 32 to 43, wherein the composition comprises less than about 1 percent by weight of fluvastatin sodium anti-isomer.

45. The stable controlled release pharmaceutical composition according to any of claims 32 to 43, wherein the composition comprises less than about 0.5 percent by weight of the fluvastatin sodium anti-isomer.

46. The stable controlled release pharmaceutical composition according to any of claims 32 to 45, wherein the composition comprises less than about 1 percent by weight of fluvastatin hydroxyl diene.

47. The stable controlled release pharmaceutical composition according to any of claims 32 to 45, wherein the composition comprise less than about 0.5 percent by weight of the fluvastatin hydroxyl diene.

48. The stable controlled release pharmaceutical composition according to any of claims 32 to 43, wherein the composition comprise less than about 1 percent by weight of either fluvastatin sodium anti-isomer or fluvastatin hydroxyl diene, following storage for three months at a temperature of 40°C and a relative humidity of 75 percent.

49. The stable controlled release pharmaceutical composition according to any of claims 32 to 43, wherein the composition comprise less than about 0.5 percent by weight of either fluvastatin sodium anti-isomer or fluvastatin hydroxyl diene, following storage for three months at a temperature of 40°C and a relative humidity of 75 percent.

50. The stable controlled release pharmaceutical composition according to any of claims 32 to 43, wherein the composition contains less than about 0.2 percent by weight of impurities and degradation products other than fluvastatin sodium anti-isomer and fluvastatin hydroxyl diene.

51. The stable controlled release pharmaceutical composition according to any of claims 32 to 43, wherein the composition contains less than about 0.1 percent of impurities and degradation products others than fluvastatin sodium anti-isomer and fluvastatin hydroxyl diene.

52. The stable controlled release pharmaceutical composition according to any of claims 32 to 43, wherein the composition contains less than about 0.2 percent of impurities and degradation products other than fluvastatin sodium anti-isomer and fluvastatin hydroxyl diene, following storage of the composition for 3 months at a temperature of 40°C and a relative humidity of 75 percent.

53. The stable controlled release pharmaceutical composition according to claim 52, wherein the composition contains less than about 0.1 percent of impurities and degradation products other than fluvastatin sodium anti-isomer and fluvastatin hydroxyl diene, following storage of the composition for 3 months at a temperature of 40°C and a relative humidity of 75 percent.

54. A stable controlled-release pharmaceutical composition, comprising fluvastatin or a salt thereof and greater than 3.5 percent by weight water.

55. The composition according to claim 54, comprising from about 10 to about 50 percent by weight of fluvastatin or a salt thereof.

56. The composition according to either of claims 54 and 55, further comprising at least one hydrophilic polymer, wherein the hydrophilic polymer is not hydroxypropyl methylcellulose, and the composition is substantially free of hydroxypropyl methyl cellulose, preferably the hydrophilic polymer is a non-ionic hydrophilic polymer.

57. The composition according to claim 56, wherein the fluvastatin is present in an amount of from about 10 to about 50 percent by weight, the at least one hydrophilic polymer is present in an amount of from about 5 to about 40 percent, and the controlled-release composition further comprises from about 20 to about 70 percent microcrystalline cellulose, from 0 to about 40 percent cross-linked polyvinyl pyrollidone, and from about 0.5 to about 2 percent of a lubricant.

58. The stable controlled release pharmaceutical composition according to any of claims 54 to 57, wherein the fluvastatin or a salt thereof has an assay purity of more than about 95 percent by weight.

59. The stable controlled release pharmaceutical composition according to any of claims 54 to 57, wherein the fluvastatin or a salt thereof has an assay purity of more than about 95 percent, following storage for three months at a temperature of 40°C and a relative humidity of 75 percent.

60. The stable controlled release pharmaceutical composition according to any of claims 54 to 59, wherein the composition comprises less than about 1 percent by weight of either fluvastatin sodium anti-isomer or fluvastatin hydroxyl diene.

61. The stable controlled release pharmaceutical composition according to any of claims 54 to 59, wherein the composition comprises less than about 0.5 percent by weight of either the fluvastatin sodium anti-isomer or fluvastatin hydroxyl diene.

62. The stable controlled release pharmaceutical composition according to any of claims 54 to 59, wherein the composition comprise less than about 1 percent by weight of either fluvastatin sodium anti-isomer or fluvastatin hydroxyl diene, following storage for three months at a temperature of 40°C and a relative humidity of 75 percent.

63. The stable controlled release pharmaceutical composition according to any of claims 54 to 59, wherein the composition comprise less than about 0.5 percent by weight of either fluvastatin sodium anti-isomer or fluvastatin hydroxyl diene, following storage for three months at a temperature of 40°C and a relative humidity of 75 percent.

64. The stable controlled release pharmaceutical composition according to any of claims 54 to 59, wherein the composition contains less than about 0.2 percent by weight of impurities and degradation products other than fluvastatin sodium anti-isomer and fluvastatin hydroxyl diene.

65. The stable controlled release pharmaceutical composition according to any of claims 54 to 59, wherein the composition contains less than about 0.1 percent of impurities and degradation products others than fluvastatin sodium anti-isomer and fluvastatin hydroxyl diene.

66. The stable controlled release pharmaceutical composition according to any of claims 54 to 59, wherein the composition contains less than about 0.2 percent of impurities and degradation products other than fluvastatin sodium anti-isomer and fluvastatin hydroxyl diene, following storage of the composition for 3 months at a temperature of 40°C and a relative humidity of 75 percent.

67. The stable controlled release pharmaceutical composition according to any of claims 54 to 59, wherein the composition contains less than about 0.1 percent of impurities and degradation products other than fluvastatin sodium anti-isomer and fluvastatin hydroxyl diene, following storage of the composition for 3 months at a temperature of 40°C and a relative humidity of 75 percent.

68. A process for preparing a pharmaceutical composition according to any of claims 32 to 67, comprising combining fluvastatin or a salt thereof with a pharmaceutical excipient.

69. A method for manufacturing a controlled release tablet, comprising granulating, drying, mixing, and compressing the composition of any of claims 1 to 29 into a tablet, and film coating the tablet.

70. A method for manufacturing the composition according to claim 69, wherein the granulation comprises wet granulation.

71. A method for manufacturing a stable tablet, comprising the steps of granulation, drying, mixing, compressing the composition of any of claims 32 to 53 into a tablet, and film coating the tablet.

72. A method for manufacturing the composition according to claim 71, wherein the granulation comprises wet granulation.

73. A method for manufacturing a stable tablet, comprising the steps of granulation, drying, mixing, compressing the composition of any of claims 54 to 67 into a tablet, and film coating the tablet.

74. A method for manufacturing the composition according to claim 73, wherein the granulation comprises wet granulation.

75. A pharmaceutical composition according to any of claims 1 to 30 and 32 to 67 for use in lowering cholesterol, preferably for use in the treatment of hyperlipoproteinemia and/or atherosclerosis.
